# EUROPEAN PATENT APPLICATION

(11) **EP 3 591 076 A1**
(43) Date of publication of application: **08.01.2020**
(21) Application number: 18460038.5
(22) Date of filing: 02.07.2018
(51) Int. Cl.: C12Q 1/6886

(54) **PRIMERS FOR DETECTING A GERMINAL MUTATION PREDISPOSING TO TYPE 1 NEUROFIBROMATOSIS AND USE OF THE SAME**

(71) Applicant: Centrum Badan DNA Spolka z Ograniczona Odpowiedzialnoscia, 61-132 Poznan (PL)
(72) Inventor: The designation of the inventor has not yet been filed
(74) Representative: Malewska, Ewa

(57) **Abstract**

The present invention relates to primers for the genotyping of a germinal mutation predisposing to developing type 1 neurofibromatosis, which form a pair composed of: a forward primer - Primer F (NF1_F), and a reverse primer - Primer R (NF1_R), having the following sequences:
a) Primer F (NF1_F): GCTCTAGACTAAGTTGCTTTCAAGTG
b) Primer R (NF1_R): ATGGGATCAATCTACTGCCTAAGA (TCTTAGGCAGTAGATTGATCCCAT)
wherein symbols A, C, G, T have their usual meaning and define individual nucleotides having the ability to amplify the sequence of the *NF-1* gene comprising variant NM_001042492.2: c.2409+1G>C, by PCR. The primers according to the invention meet the following basic criteria: they are 26 and 24 bases long (F = 26 bases, R = 24 bases respectively), have a melting point in the range 58-60°C and the ratio of GC to AT bases suitable for oligonucleotides that are primers for sequence amplification.

The invention also encompasses the use of primers as defined above in genetic tests for the amplification of germinal mutation sequences predisposing to developing type 1 neurofibromatosis, by PCR.

## Description

The invention relates to new primers for genotyping of a previously non-described germinal mutation predisposing to developing type 1 neurofibromatosis and to employing a methodology of identification of the mutation in genetic tests identifying predisposition to develop type 1 neurofibromatosis.

The present invention is of a crucial importance in diagnostics of type 1 neurofibromatosis. Due to the high variability of clinical symptoms and some cases of mild expression, diagnosis of the disease is frequently delayed. Type 1 neurofibromatosis (NF1, OMIM: # 162200), also known as Recklinghausen disease, is one of the most common genetic diseases; its frequency, depending on the population under investigation, is estimated at 1: 2,500 to 1: 3,000 births. NF1 is inherited in an autosomal dominant manner with a penetration of up to 100% and a highly variable expression of the NF1 gene mutation coding for neurofibromine 1 (2, 9). Half of the cases are due to new and not inherited mutations, however children of such people inherit the disease with a typical risk of 50%.

Type 1 neurofibromatosis belongs to the group of fakomatoz - a group of developmental disorders of tissues from three germ layers. The course of the disease shows variation in the severity of symptoms. In the clinical picture of the disease there are skin and eye lesions, intracranial tumors and other noncancerous locoregional tumors, as well as bone lesions. In some patients mild course of the disease is observed, while in others it may occur with significant intensity, which causes a marked impairment of the quality of life of patients. Neurofibromatosis is a disease that significantly reduces the quality of life as well as the survivorship of patients. The external appearance of patients is often changed by the occurrence of multiple nodules, the so-called neurofibromas, which are benign neoplastic lesions. The resulting neurofibromas originate from peripheral nerves and when they develop near the spinal canal they can cause compression of the spinal cord and paralysis, as a result. In addition, in some patients neurofibromas may develop towards malignant tumors. Patients with NF1 also have an increased risk of other benign neoplastic lesions but also malignant ones. Central nervous system tumors, such as optic nerve glioma and neurofibromas of the spinal cord, are common. The most frequently transformed into malignant neoplasms are convolutional neurofibromas, from which malignant peripheral nerve envelope (MPNST) tumors originate. Patients with NF1 also have rhabdomyosarcomas and leukemia.

Diagnostics of type 1 neurofibromatosis is a serious clinical problem - in a significant number of cases the disease remains undiagnosed. Creating a diagnostic test based on DNA analysis of patients is therefore crucial to improve the quality of diagnosis of the disease.

The c.2409+1G>C change was classified as a variant with an unknown clinical effect, possibly a pathogenic one. It occurs in the splice site, which is a conservative region. The detected variant was classified by the MutationTaster program as a disease causing. This variant was detected for the first time in our internal database. The frequency of this variant - MAF (minor allele frequency) for the global population according to the base 1000Genomes, is 0. The change c.2409+1G>C has not been thus far described in the scientific literature, however, another variant of c.2409+1G>A, in the same position on the chromosome, was recorded in the HGMD Professional (Human Gene Mutation Database) as a disease causing (pathological) mutation correlated with neurofibromatosis type 1. According to EMQN - European Molecular Genetics Quality Network, guidelines regarding the classification of variants, changes not described in the literature found - however, in the same position in the chromosome as the variants regarded as pathogenic, are considered to be of a significant clinical relevance.

The essence of the invention

Primers for PCR amplification of the NF-1 gene sequence containing the tested variant NM_001042492.2: c.2409+1G>C, described herein as a variant of a potentially significant clinical relevance, are paired: the forward primer - Primer F (NF1_F), and the reverse primer - Primer R (NF1_R), and have the following sequences:
1. Primer F (NF1_F): GCTCTAGACTAAGTTGCTTTCAAGTG
2. Primer R (NF1_R):ATGGGATCAATCTACTGCCTAAGA (TCTTAGGCAGTAGATTGATCCCAT)
The symbols A, C, G, T define individual nucleotides.

The primers of the invention are characterized by a complementarity to a particular fragment of the DNA sequence that includes the sought c.2409+1G>C change and enable the amplification of this fragment in the PCR reaction environment, and thus make it possible to detect the change under study.

**Primer pair 1**

| | | | | | | | | | | | |
|---|---|---|---|---|---|---|---|---|---|---|---|
| | **Sequence (5'->3')** | | | **Template strand** | **Length** | **Start** | **Stop** | **Tm** | **GC%** | **Self complementarity** | **Self 3' complementarity** |
| **Forward primer** | GCTCTAGACTAAGTTGCTTTCAAGTG | | | Plus | 26 | 15 | 40 | 60.13 | 42.31 | 6.00 | 2.00 |
| **Reverse primer** | ATGGGATCAATCTACTGCCTAAGA | | | Minus | 24 | 679 | 656 | 58.84 | 41.67 | 4.00 | 2.00 |
| **Product length** | 665 | | | | | | | | | | |
| | | | | | | | | | | | |
| **Products on potentially unintended templates** | | | | | | | | | | | |
| >NC_000017.11 Homo sapiens chromosome 17. GRCh38.p7 Primary Assembly | | | | | | | | | | | |
| | | | | | | | | | | | |
| product length = 665 | | | | | | | | | | | |
| Features associated with this product: | | | | | | | | | | | |
| neurofibromin isoform 1 | | | | | | | | | | | |
| neurofibromin isoform 2 | | | | | | | | | | | |
| Forward primer | 1 | GCTCTACACTAA5TTGCTTTCAAGTG | 26 | | | | | | | | |
| Template | 31227478 | .......................... | 31227503 | | | | | | | | |
| Reverse primer | 1 | ATGGGATCAATCTACTGCCTAAGA | 24 | | | | | | | | |
| Template | 31228142.... | .................... | 31228119 | | | | | | | | |

The primers according to the invention meet the following basic criteria:
1. length of 26 and 24 bases (respectively F = 26 bases, R = 24 bases),
2. melting point in the range of 58-60°C
3. the proportion of GC to AT bases typical for oligonucleotides that are primers for the amplification of DNA sequences.

The invention also relates to the use of the primers as defined above in a PCR reaction for the amplification of the *NF1* gene sequence describing the site of the germinal mutation that contributes to the onset of type 1 neurofibromatosis.

In combination with the New Generation Sequencing (NGS) technology, the solution according to the present invention allows also for a possibility of analyzing the full gene sequence, reduces the cost of the single mutation analysis and also give the possibility of high-throughput multiplexing of samples. Such a procedure considerably improves molecular diagnostics.

### Detail description of the invention

In the course of work on the present solution, the latest techniques were used to develop the primers according to the invention and a genetic test for determining germinal mutations using the specific primers, significantly improving the genotyping of a given mutation in a patient.

DNA was isolated from the collected biological material using the inorganic method. The quality and purity of the isolated genetic material was determined by electrophoretic separation and by spectrophotometric methods. The concentration of DNA was determined by the fluorymetric method. Direct sequencing was carried out using the Illumina MiSeq sequencer with a reading of 2 x 150 bp. Sequence read-outs were compared to the human reference genome hgl9 (37.1) using the MiSeq Reporter program. Genetic tests were performed within > 95% of coding regions and flanking intron sequences contained in the *NF1* gene. As a result, it was possible to detect single nucleotide substitutions and small deletions/insertions.

The employed NGS sequencing technology, i.e. Next Generation Sequencing, is based on the method of sequencing by reversible termination. This is a very efficient method for genotyping DNA sequences, because the sequencing takes place on the MiSeq platform (Illumina), which allows reading up to 15 GB (15x10⁹ bases) from one sequencing in 45 million paired readings. This method assumes hybridization of previously fragmented genomic DNA on a solid support. Sequence reading is done by simultaneous sequencing of many fragments. The nucleotides present in the reaction mixtures that are labeled with fluorescent dyes and have the ability of stopping the reactions reversibly, are attached successively to the elongated sequence. After binding of a proper nucleotide, the reaction stops. The remaining nucleotides in the mixture are washed away. The laser light excites the dye and the reading takes place. After that the chemical group that blocks the reaction is removed and it is possible to attach the next nucleotide. Due to the presence of all four nucleotides with an attached reversible terminator during each sequencing cycle, natural competition minimizes embedding errors.

The end result is the nucleotide sequencing that allows to obtain the most accurate data in a wide range of the application. The technology based on the sequencing of the next generation NGS requires a much smaller amount of template DNA (nanograms), and the DNA test allows for an analysis of even more than several thousand nucleotide changes in one study.

The NGS technology has a number of advantages from the point of view of the solution according to the invention, such as among the others:
- the current genetic test for determination of *NF1* gene mutations is based on a technology that allows the whole gene to be sequenced during a single analysis,
- accuracy of the tests performed is higher than 99.9% for a single base, i.e. it is incomparably higher than in other laboratory methods,
- this technology allows for the analysis of the most important region of the analyzed gene, guaranteeing the highest diagnostic effectiveness,
- the genetic test is non-invasive and painless - just cheek swab or peripheral blood, with peripheral blood being the preferred material.

Genetic research in NGS technology represents a huge advance in medicine - fast and accurate diagnostics results in an effective prophylaxis and treatment. NGS technology depends to a large extent on IT systems both in the area of data processing devices (computers with parameters that allow to process growing databases - very high rate of data increase as a result of increasing the product scope in the form of simultaneously tested number of DNA) and in software allowing analysis of research results (the process of improving programs and/or creating completely new ones).

The present invention relates to the method of sequence analysis of *NF1* gene alleles responsible for the occurrence of the mutation resulting in development of type 1 neurofibromatosis. Primers according to the invention for amplifying the *NF1* gene sequence allow identification of a variant of the gene encoding the neurofibrominase 1 protein whose heterozygous mutations cause type 1 neurofibromatosis.

The primers of the invention are used in a genetic test enabling genotyping of the germinal mutation of the *NF1* gene, which comprises three steps of the laboratory procedure:
- PCR - polymerase chain reaction,
- NGS sequencing,
- bioinformatic analysis of results.

PCR - polymerase chain reaction.

The primers of the invention are used to amplify well-defined DNA regions including the *NF1* gene. The following ares used for the reaction: the DNA fragment to be amplified, a mixture of nucleotides, thermostable DNA polymerase and the primers designed appropriately for the studied change, i.e. short DNA fragments complementary to the fragments of the template of the amplified gene. There are two types of primers: Primer F (forward), the sequence of which is the same as the duplicated sequence and Primer R (reverse), the sequence of which is complementary to the duplicated one. In this way, they form a pair of primers suitable for amplification of well-defined regions of the template DNA.

Amplification is carried out separately for each analyzed sample and is a cycle of repeated three stages of reaction: denaturation, annealing and elongation, i.e. the synthesis of a new DNA chain. Each one of the stages takes place at a strictly defined temperature, depending on the primers used, reagents included in the reaction mixture and the amplified DNA fragment.

The presence of amplicons is then confirmed by agarose gel electrophoresis in the presence of appropriate DNA length markers, which additionally enable verification of the length of the PCR products obtained.

**Table 1. The length of amplicons for a pair of primers used to analyze the germinal mutation c.2409+1G>C of the NF1 gene**

| Gene | The name of the primer | The primer's sequence | Amplicon length |
|---|---|---|---|
| NF1 | NF1_F | GCTCTAGACTAAGTTGCTTTCAAGTG | 665 |
| | NF1_R | ATGGGATCAATCTACTGCCTAAGA | |

### NGS sequencing:

The resulting amplicons are then used to prepare the so-called NGS libraries, by enzymatic fragmentation of the DNA sample into small segments, then joining them with the adapter sequences (indexing) and purifying using microreactor magnetic beads. The final stage is to perform sequencing by synthesis, which allows to obtain the genotyping result of the *NF1* gene at the elevated or high resolution level.

Wykorzystywane tu sekwencjonowanie przez syntez 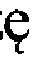 (ang. *sequencing by synthesis,* SBS) jest obecnie uwazane za najskuteczniejsze i w konsekwencji jest to najszerzej stosowana technologia w platformach sekwencjonowania nast 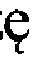 pnej generacji (NGS). Technologia zak 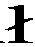 ada hybrydyzacj 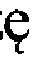 DNA na stalym podlozu (flow-cell), a odczyt sekwencji odbywa si 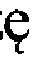 jednoczesnie dla wielu fragmentów. Obecne w mieszaninie reakcyjnej wyznakowane barwnikami fluorescencyjnymi nukleotydy posiadaj 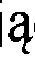 ce cech 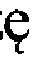 odwracalnego zatrzymywania reakcji, przy 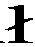 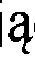 czaj 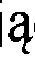 si 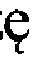 do wyd 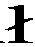 użaj 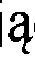 cej si 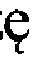 sekwencji. Po zwi 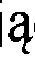 zaniu si 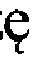 odpowiedniego nukleotydu reakcja zatrzymuje si 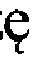 , a pozostale nukleotydy zostaj 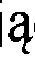 wyp 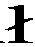 ukane z mieszaniny. Nast 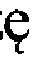 pnie swiatlo lasera wzbudza barwnik i nast 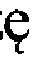 puje odczyt. Blokuj 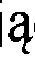 ca reakcj 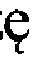 grupa chemiczna jest usuwana i możliwe jest przy 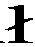 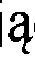 czenie kolejnego nukleotydu

Sequencing by synthesis (SBS) used in the present studies is currently considered the most effective and consequently the most widely used technology in the next-generation sequencing platforms (NGS). The technology assumes hybridization of DNA on a solid base (flow-cell), and the sequence reading takes place simultaneously for many fragments. The nucleotides present in the reaction mixture, labeled with fluorescent dyes and having the property of reversibly stopping the reaction, are attached to the elongated sequence. Once the appropriate nucleotide has been bound, the reaction stops and the remaining nucleotides are washed out of the mixture. Then the laser light excites the dye and the reading takes place. The chemical group blocking further reaction is then removed and it is possible to attach next nucleotide (Pi 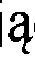 tkowski J., Skalniak A., Bodzioch M., Pach D., Hubalewska-Dydejczyk A., "Introduction to human genome sequencing", Przegl 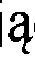 d Lekarski 2013/70/7 and Profaizer T, Lázár-Molnár E, Close DW, Delgado JC, Kumánovics A., "HLA genotyping in the clinical laboratory: comparison of next-generation sequencing methods.", HLA. 2016 Jul; 88(1-2): 14-24, Pubmed ID: 27524804).

### Bioinformatic analysis of results:

In the first step, raw readings in the FASTQ format are subjected to quality control using Trimmomatic software - version 0.35 (Bolger, AM, Lohse, M., & Usadel, B. (2014).) Trimmomatic: A flexible trimmer for Illumina Sequence Data. btu170). Nucleotides of average quality, less than 15 in a 4 nucleotide window, are removed from the set of data. In addition, readings less than 30 bp are also removed. The thus prepared FASTQ files are subjected to mapping (Li H. and Durbin R. (2009) Fast and accurate short read alignment with Burrows-Wheeler Transform. Bioinformatics, 25:1754-60. [PMID: 19451168]).

As indicated above, conducting the PCR reaction requires proper selection of parameters: appropriate reaction conditions, primers for the amplification reaction and selection of components of the reaction mixture. As it results from the above description, to carry out the PCR reaction, it is not necessary to know the sequence of the tested gene, only the knowledge of the nucleotide sequence in the sections flanking the examined change is sufficient.

The most important criteria for the selection of primers according to the invention, and enabling their use in the genotyping of the *NF1* gene are: primer lengths of 20-30 bases, melting temperatures between 58-61°C and a suitable ratio of GC to AT bases.

Table 2 below lists the properties of the primer according to the invention, calculated using the Multiple Primer Analyzer available at: (https://www.thermofisher.com/pl/en/home/brands/thermo-scientific/molecular-biology/ molecular-biology-leaming-center/molecular-biology-resource-library/thermo-scientific-web-tools/multiple-primer-analyzer.html).
Table 2. Sekwencje starterów oraz ich w 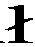 aściwości

| Gene | The primers' sequences | Tm°C | GC% | Estimation of the product length (bp) |
|---|---|---|---|---|
| NF1 | GCTCTAGACTAAGTTGCTTTCAAGTG | 60,13 | 42,31 | 665 |
| | ATGGGATCAATCTACTGCCTAAGA | 58,84 | 41,67 | |

### Example.

Using the inorganic method, DNA was isolated from the biological material collected for the study. The quality and purity of the isolated genetic material was determined by electrophoretic separation and by spectrophotometric method. The concentration of DNA was determined by the fluorymetric method. Direct sequencing was carried out using the Illumina MiSeq sequencer with a reading of 2 x 150 bp. Sequence read-outs were compared to the human reference genome hg19 (37.1) using the MiSeq Reporter program. Genetic tests were performed within > 95% of coding regions and flanking intron sequences contained in the *NF1* gene.

As a result, it was possible to detect single substitutions of nucleotides and small deletions/insertions, responsible for the increased susceptibility to type 1 neurofibromatosis.

The identification of a germinal mutation predisposing to developing type 1 neurofibromatosis allows for the use of an appropriate therapy at an earlier stage than in the state of the art.

## Claims

1. Primers for the genotyping of a germinal mutation predisposing to developing type 1 neurofibromatosis, which form a pair composed of: a forward primer - Primer F (NF1_F), and a reverse primer - Primer R (NF1_R), having the following sequences:
a) Primer F (NF1_F): GCTCTAGACTAAGTTGCTTTCAAGTG
b) Primer R (NF1_R): ATGGGATCAATCTACTGCCTAAGA (TCTTAGGCAGTAGATTGATCCCAT)
wherein symbols A, C, G, T have their usual meaning and define individual nucleotides having the ability to amplify the sequence of the *NF-1* gene comprising variant NM_001042492.2: c.2409+1G>C, by PCR.

2. Primers according to claim 1, **characterized in that** they fulfill the following basic criteria: length of 26 and 24 bases (respectively Primer F = 26 bases, Primer R = 24 bases), melting point in the range 58-60°C and the ratio of GC to AT bases appropriate for a primer oligonucleotides for amplification of DNA sequences.

3. The use of the primers set forth in claim 1, in genetic tests for amplification by PCR of germinal mutation sequences predisposing to developing type 1 neurofibromatosis.


<110>  CENTRUM BADAN DNA Spolka z ograniczona odpowiedzialnoscia.

<120>  PRIMERS FOR DETECTING A GERMINAL MUTATION PREDISPOSING
       TO TYPE 1 NREUROFIBROMATOSIS AND USE OF THE SAME
       (Startery do wykrywania mutacji germinalnej predysponuj¹cej do
       zachorowañ na neurofibromatozê typu 1 oraz ich zastosowanie)

<130>  I/212600.ep

<140>  not known yet
<141>  2018-07-02

<160>  3

<170>  PatentIn version 3.3


<210>  1
<211>  26
<212>  primer 1 (forward)

<400>  1
GCTCTAGACTAAGTTGCTTTCAAGTG                              26


<210>  2
<211>  24
<212>  primer 2

<400>  2
ATGGGATCAATCTACTGCCTAAGA                                                    24


<210>  3
<211>  22
<212>  primer 3

<400>  3
TCTTAGGCAGTAGATTGATCCCAT                                                  24